# EUROPEAN PATENT SPECIFICATION

(11) **EP 3 305 165 B1**
(45) Date of publication and mention of the grant of the patent: **14.08.2019**
(21) Application number: 16800029.7
(22) Date of filing: 24.05.2016
(51) Int. Cl.: A61B 1/00

(54) **MEDICAL TUBE AND MEDICAL SYSTEM**
MEDIZINISCHER TUBUS UND MEDIZINISCHES SYSTEM
TUBE MÉDICAL ET SYSTÈME MÉDICAL

(30) Priority: 25.05.2015 JP 2015105609
(43) Date of publication of application: 11.04.2018
(73) Proprietor: Olympus Corporation, Hachioji-shi Tokyo 192-8507 (JP)
(72) Inventor: HIGUCHI, Tatsuya, Hachioji-shi Tokyo 192-8507 (JP)
(74) Representative: Schicker, Silvia
(86) International application number: PCT/JP2016/065362
(87) International publication number: WO 2016/190324

(56) References cited:
- WO-A2-2013/144914
- JP-A- 2001 299 684
- JP-A- 2008 538 709
- JP-A- 2013 172 780
- US-A- 5 025 778
- US-A1- 2010 063 358
- US-A1- 2011 315 147

## Description

### {Technical Field}

The present invention relates to a medical tube and a medical system.

### {Background Art}

In the related art, there is a known tube provided with a plurality of lumens into which an endoscope and a treatment tool are inserted (for example, see Patent Literature 1).

The tube of Patent Literature 1 has a lumen that can be expanded in order to ensure a large enough channel for inserting a treatment tool or the like while making the tube diameter small during insertion. Further document US 5 025 778 A A1 discloses a medical manipulator system as set out in the preamble of claims 1 or 4.

### {Citation List}

### {Patent Literature}

{Patent Literature 1} Japanese Translation of PCT International Application, Publication No. 2008-538709

### {Summary of Invention}

### {Technical Problem}

However, because the tube is formed of a material possessing high flexibility in order to allow bending thereof in conformity to winding pathways in the body, the torque transmittability thereof is low, and thus, there is a problem in that it is not possible to rotate the distal end of the tube about the longitudinal axis even if the proximal end of the tube is twisted outside the body after inserting the tube into the body. In other words, there is a problem in that, at a desired position in an image acquired by using the endoscope inserted into one channel of the tube, it is not possible to dispose a treatment tool or the like inserted via another channel.

The present invention has been conceived in light of the above-described circumstances, and an object thereof is to provide a medical tube and a medical system with which it is possible, while maintaining high flexibility, to make a treatment tool protrude at an appropriate position with respect to an endoscope.

### {Solution to Problem}

In order to achieve the above-described object, the present invention provides the following solutions.

An aspect of the present invention is a medical tube including: a first lumen that forms an endoscope channel by penetrating through a tube main body, which is constituted of a material possessing flexibility, in a longitudinal-axis direction; a plurality of second lumens that are capable of forming treatment-tool channels by penetrating through the tube main body in the longitudinal-axis direction at a periphery of the first lumen with spacings therebetween in a circumferential direction; a first indicator that is provided on an inner surface in the vicinity of a distal end of the first lumen and that specifies a circumferential-direction position of any one of the second lumens with respect to the first lumen; and a second indicator that is provided at a proximal end of the tube main body, and that indicates the second lumen specified by the first indicator.

With this aspect, when an image is captured by operating the endoscope while inserting the endoscope by using the first lumen of the tube main body as the endoscope channel, an image in which the first indicator provided on the inner surface in the vicinity of the distal end of the first lumen is captured is acquired. Therefore, in order to make a treatment tool protrude from the distal-end opening of any one of the second lumens specified by the first indicator in the image, an operator can easily check, by means of the second indicator at the proximal end of the tube main body, the proximal-end opening of the second lumen into which the treatment tool should be inserted. In other words, even if the tube main body is distorted about the longitudinal axis of the endoscope, it is possible to make the treatment tool protrude from a desired position with respect to the endoscope without correcting the distortion by means of torque.

In the above-described aspect, the second lumens may be provided so that inner diameters thereof can be expanded in directions in which an outer diameter of the tube main body would be increased.

With this configuration, in the second lumens in the state in which the treatment tool is not inserted, the outer diameter of the tube main body is decreased by contracting the inner diameters of the second lumens, and thus, it is possible to enhance the ease of inserting the medical tube into the body. In addition, by expanding the inner diameter of only the second lumen to be used as the treatment tool channel, it is possible to minimize the increase in the outer diameter of the tube main body.

The above-described aspect may be provided with a cap member that is attached to the proximal end of the tube main body, wherein the cap member may have a large enough diameter to allow a treatment tool to be inserted thereinto, and may be provided with a plurality of insertion cylindrical portions that are inserted into proximal-end openings of the individual second lumens and expand the proximal-end openings.

With this configuration, by expanding portions in the vicinity of the proximal-end openings of the second lumens by individually inserting the plurality of insertion cylindrical portions of the cap member into the proximal-end openings of the plurality of second lumens, it is possible to easily insert the treatment tool into the second lumen via the insertion cylindrical portion.

In the above-described aspect, the second indicator may be provided on the cap member.

With this configuration, when inserting the treatment tool into the second lumen from the cap member, which is attached to the most proximal end of the tube main body, it is possible to easily perform the insertion by using the second indicator provided on the cap member as markers.

In the above-described aspect, the first indicators may be identification information of the individual second lumens with which the second indicators corresponding thereto can be identified.

By doing so, it is possible to identify the second lumen itself by using the first indicator constituted of the identification information acquired by using the endoscope, and, by inserting the treatment tool into the proximal-end opening of the second lumen in accordance with the corresponding second indicator, it is possible to easily make the treatment tool protrude at the desired position in the image. For example, the first indicator may be a set of numbers representing the individual second lumens; and the operator can read a number specified by the first indicator in the image, and thus, he/she can insert the treatment tool into the second lumen from the proximal-end opening to which the second indicator corresponding to said number is assigned.

Another aspect of the present invention is a medical system including: any one of the above-described medical tubes; an endoscope that is inserted into the first lumen of the medical tube; a display portion that displays an image acquired by using the endoscope; an image-processing portion that recognizes the first indicator in the image; and a notifying portion that issues, on the basis of the first indicator recognized by the image-processing portion, a notification about the identification information of one or more of the second lumens specified by the first indicator.

With this aspect, when the distal end of the medical tube is placed in the body of the patient and when the endoscope is inserted into the endoscope channel formed of the first lumen, an image that includes the first indicator provided on the inner surface of the first lumen is acquired, and thus, the first indicator is recognized by the image-processing portion. Because a notification about the identification information of the second lumen is issued by the notifying portion on the basis of the recognized first indicator, the operator can easily ascertain, on the basis of the content of the notification, the second lumen into which the treatment tool should be inserted.

In the above-described aspect, the notifying portion may display, in the image displayed on the display portion, the identification information in association with positions of distal-end openings of one or more of the second lumens.

With this configuration, just by inserting the treatment tool into the second lumen from the proximal-end opening that corresponds to the second indicator indicated by the identification information corresponding to the position of the desired distal-end opening displayed in the image, the operator can make the treatment tool protrude from the desired distal-end opening in a simple manner.

In the above-described aspect, the notifying portion may issue, on the basis of the first indicator recognized by the image-processing portion, a notification about the identification information of one or more of the second lumens to be used as the treatment-tool channels.

With this configuration, just by inserting the treatment tool into the second lumen from the proximal-end opening that corresponds to the second indicator identified by the identification information about which a notification is issued by the notifying portion, it is possible to make the treatment tool protrude from the desired distal-end opening in a simple manner.

The above-described aspect may be provided with an input portion with which information about a treatment tool to be used is input, wherein the notifying portion may issue, on the basis of the information about the treatment tool input via the input portion, a notification about the identification information of one or more of the second lumens that are suitable to be used as the treatment-tool channels.

With this configuration, just by inputting the information about the treatment tool to be used via the input portion and by inserting the treatment tool into the second lumen from the proximal-end opening that corresponds to the second indicator identified by the identification information about which a notification is issued, it is possible to insert the treatment tool into the second lumen that is suitable for the treatment tool to be used.

In the above-described aspect, the notifying portion may issue a notification by making the second indicators corresponding to the second lumens to be used as the treatment-tool channels differ from the rest of the second indicators at the proximal end of the tube main body.

With this configuration, just by inserting the treatment tool from the proximal-end opening that corresponds to the second indicator that is indicated by means of the method differing from the rest, it is possible to make the treatment tool protrude at the desired position in the image.

### {Advantageous Effects of Invention}

The present invention affords an advantage in that it is possible, while maintaining a high flexibility, to make a treatment tool protrude at an appropriate position with respect to an endoscope.

### {Brief Description of Drawings}

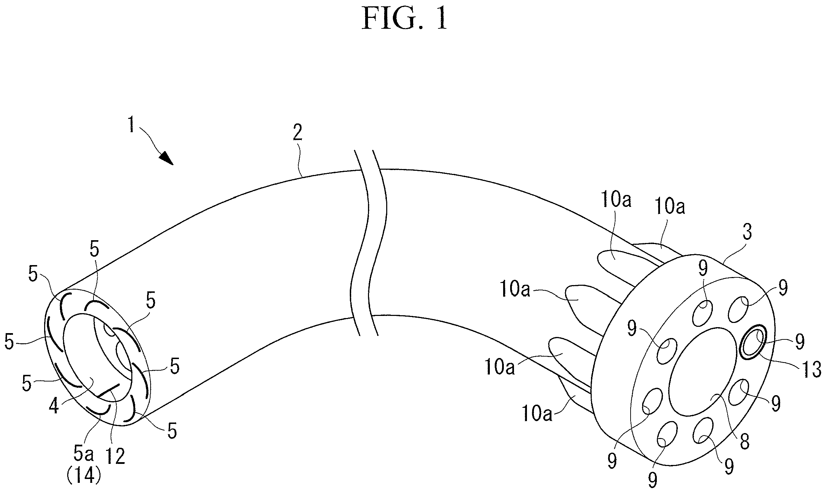

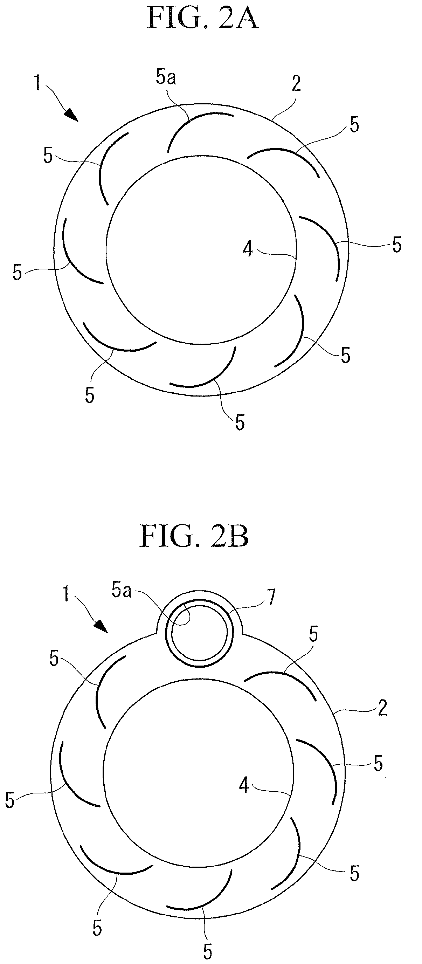

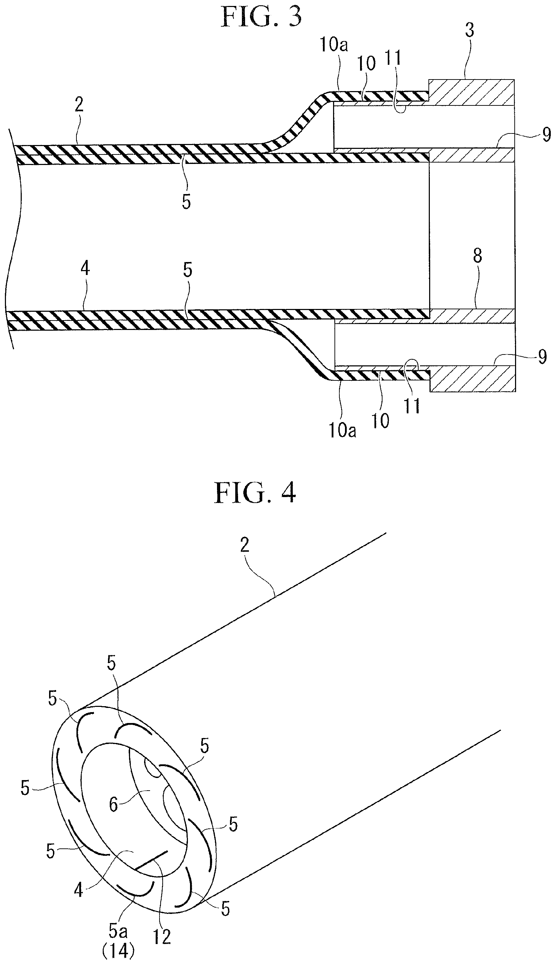

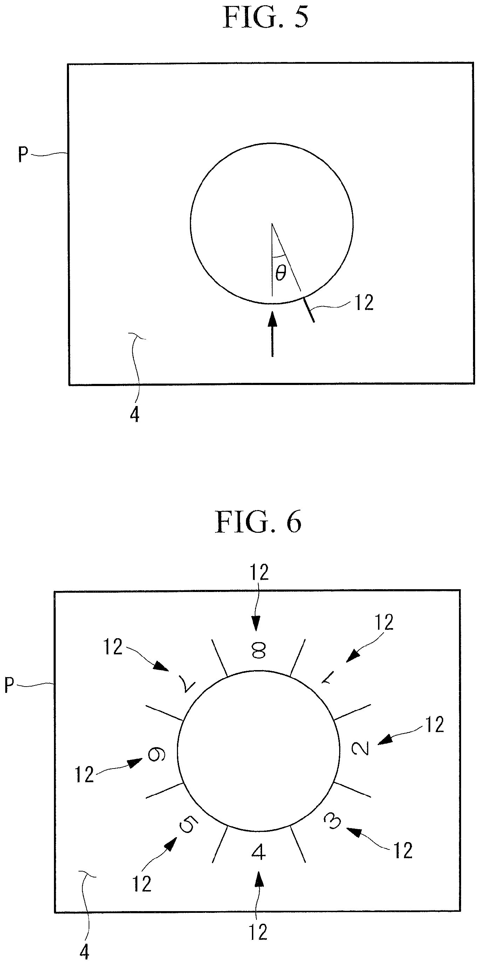

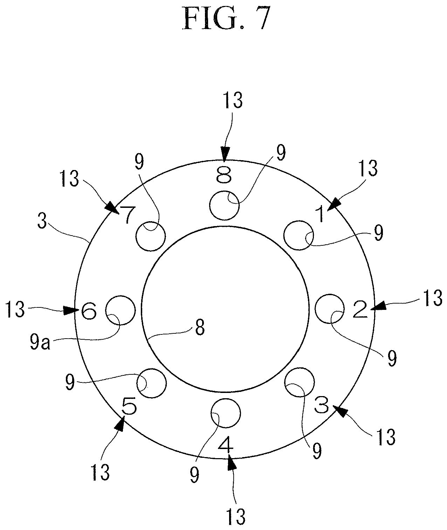

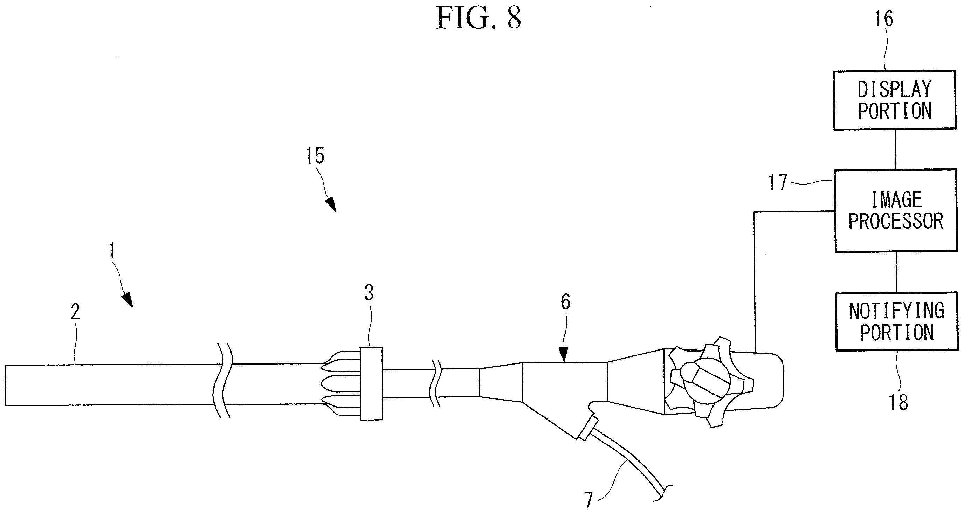

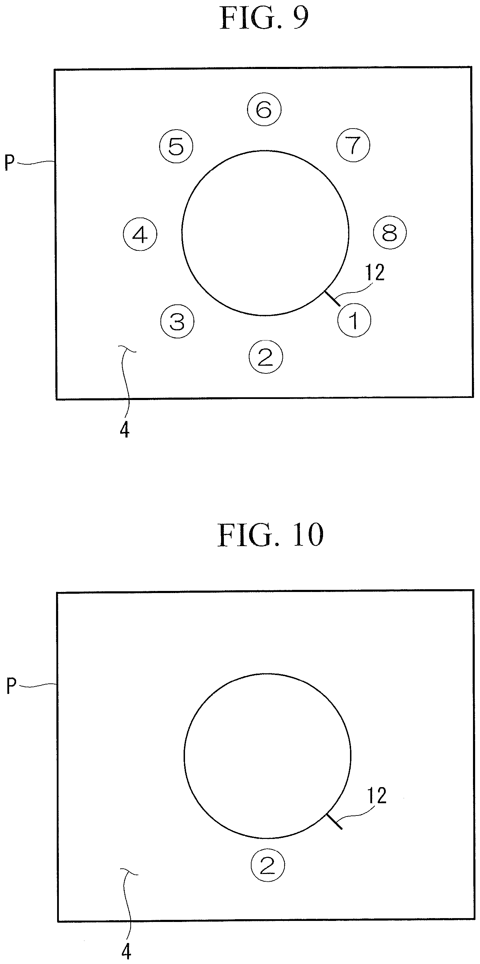

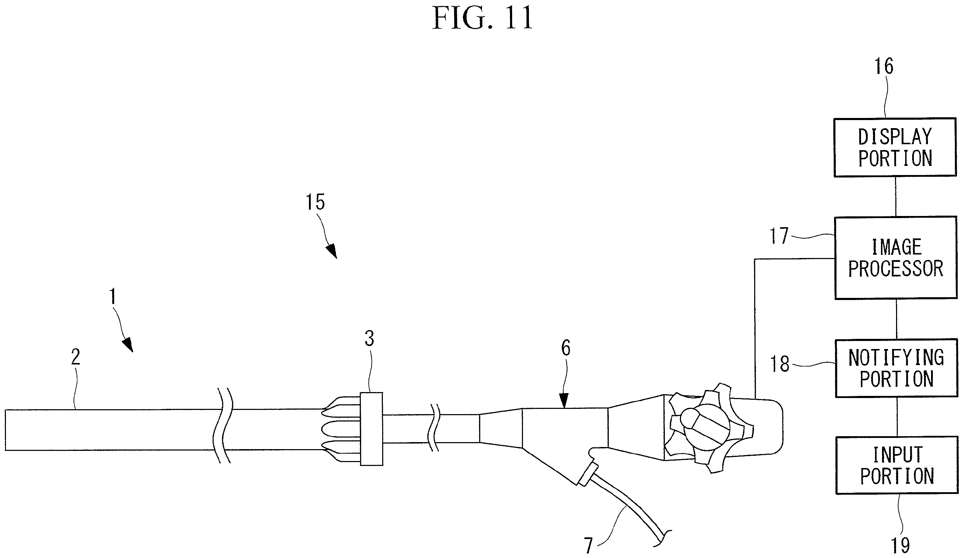

{Fig. 1} Fig. 1 is a perspective view showing a medical tube according to an embodiment of the present invention.
{Fig. 2A} Fig. 2A is a lateral cross-sectional view in which a treatment tool is not inserted into any of second lumens in the medical tube in Fig. 1.
{Fig. 2B} Fig. 2B is a lateral cross-sectional view in which the treatment tool is inserted into one of the second lumens in the medical tube in Fig. 1.
{Fig. 3} Fig. 3 is a longitudinal cross-sectional view showing a cap member attached to a proximal-end portion of the medical tube in Fig. 1.
{Fig. 4} Fig. 4 is a perspective view showing a distal-end portion of the medical tube in Fig. 1 in a state in which an endoscope is inserted into a first lumen.
{Fig. 5} Fig. 5 is a diagram showing an example of an image acquired by using the endoscope inserted into the first lumen in the medical tube in Fig. 1.
{Fig. 6} Fig. 6 is a diagram showing an example of an image that includes a modification of a distal-end marker of the medical tube in Fig. 1.
{Fig. 7} Fig. 7 is a front view of the cap member showing a modification of a proximal-end marker of the medical tube in Fig. 1.
{Fig. 8} Fig. 8 is an overall configuration diagram showing a medical system according to an embodiment of the present invention.
{Fig. 9} Fig. 9 is a diagram showing an example of an image that includes the numbers assigned to all second lumens about which a notification is issued by a notifying portion of the medical system in Fig. 8.
{Fig. 10} Fig 10 is a diagram showing an example of an image that includes the number assigned to the second lumen into which the treatment tool should be inserted, about which a notification is issued by the notifying portion of the medical system in Fig. 8.
{Fig. 11} Fig. 11 is an overall configuration diagram showing a modification of the medical system in Fig. 8.

### {Description of Embodiment}

A medical tube 1 according to an embodiment of the present invention will be described below with reference to the drawings.

As shown in Fig. 1, the medical tube 1 according to this embodiment is provided with a tube main body 2 formed of a flexible material, for example, a silicone resin, and a cap member 3 attached to a proximal end of the tube main body 2.

The tube main body 2 is provided with, at substantially the center of a lateral cross-section thereof, a first lumen 4 that passes therethrough in the longitudinal-axis direction, and a plurality of second lumens 5 and 5a that are provided at a periphery of the first lumen 4 with spacings therebetween in the circumferential direction.

The first lumen 4 has a large enough diameter to allow an endoscope 6 to be inserted thereinto and forms an endoscope channel. In the state in which a treatment tool 7 is not inserted, the second lumen 5a is collapsed, as shown in Fig. 2A, and, when the treatment tool 7 is inserted, the second lumen 5a is expanded by the treatment tool 7, as shown in Fig. 2B, thus being expanded to a diameter that allows the treatment tool 7 to be inserted thereinto so as to form a treatment-tool channel.

As shown in Fig. 2A, when the second lumens 5 and 5a are expanded, because the outer surface of the tube main body 2 is expanded radially outward, the outer diameter of the tube main body 2 is decreased to a minimum size in the state in which the second lumens 5 and 5a are collapsed, as shown in Fig. 2A.

As shown in Fig. 3, the cap member 3 is formed in an annular shape having a center through-hole 8 that has a diameter substantially equivalent to that of the first lumen 4 and that is provided at a position aligned with the first lumen 4 in a state in which the cap member 3 is attached to the proximal end of the tube main body 2. At the periphery of the center through-hole 8, as many peripheral through-holes 9 and 9a as there are the second lumens 5 and 5a are provided at positions aligned with the second lumens 5 and 5a.

The peripheral through-holes 9 and 9a each have a large enough diameter to allow the treatment tool 7 to be inserted thereinto. In addition, a plurality of insertion cylindrical portions 10 that extend in an axial direction from positions aligned with the individual peripheral through-holes 9 and 9a are provided at one end of the cap member 3 in the axial direction. As shown in Fig. 3, the cap member 3 is attached to the proximal end of the tube main body 2 by inserting the insertion cylindrical portions 10 by expanding proximal-end openings 11 of the second lumens 5 and 5a (reference sign 10a in Fig. 1 indicates portions of the tube main body 2 that are bulged out due to insertion of the insertion cylindrical portions 10 into the second lumens 5).

As shown in Fig. 1, the medical tube 1 according to this embodiment is provided with a distal-end marker (first indicator) 12 that is provided on an inner surface of the first lumen 4 in the vicinity of the distal end of the tube main body 2 and a proximal-end marker (second indicator) 13 that is provided on the cap member 3.

The distal-end marker 12 is a single straight line that is drawn along the axial direction so as to indicate a specific position in the circumferential direction. This distal-end marker 12 specifies a distal-end position of a single second lumen 5a that exists in the vicinity of the position of the distal-end marker 12.

The proximal-end marker 13 is provided at the position of any one of the peripheral through-holes 9a of the cap member 3 so as to indicate a proximal-end position of the second lumen 5a for which the distal-end position thereof is specified by the distal-end marker 12. In the example shown in Fig. 1, the proximal-end marker 13 is a circular marker that surrounds the peripheral through-hole 9a to be specified.

The operation of the thus-configured medical tube 1 according to this embodiment will be described below.

In order to introduce the endoscope 6 and the treatment tool 7 by using the medical tube 1 according to this embodiment so as to reach an affected portion in the body, the medical tube 1 is inserted into the body of a patient from the distal-end side thereof in a state in which the endoscope 6 is inserted into the first lumen 4 of the medical tube 1. At this time, as shown in Fig. 4, in a state in which the endoscope 6 is slightly retracted into the first lumen 4 from the distal end of the medical tube 1, the endoscope 6 is disposed at a position at which it is possible to acquire an image P that includes an inner surface of the first lumen 4, as shown in Fig. 5.

Because the medical tube 1 is formed of a highly flexible material and has a very small outer diameter in the state in which the treatment tool 7 is not inserted, when inserting the endoscope 6, it is possible to insert the endoscope 6 without hindering bending thereof and, in addition, without exerting a large burden on the patient. However, because the medical tube 1 does not have a high torsional rigidity as does the endoscope 6, relative distortion sometimes occurs with respect to the endoscope 6 during insertion.

In this case, with the medical tube 1 according to this embodiment, because the distal-end marker 12 provided on the inner surface of the first lumen 4 and the proximal-end marker 13 provided on the cap member 3 are provided so as to indicate the distal-end opening 14 and the peripheral through-hole (proximal-end opening) 9a of the same the second lumen 5a and, even if the medical tube 1 is distorted with respect to the endoscope 6, an operator can set, by using the markers 12 and 13 as references, the peripheral through-hole 9a from which the treatment tool 7 can be protruded to a desired position in the image P acquired by the endoscope 6.

In other words, in the image P acquired by using the endoscope 6, the operator checks the position of the distal-end marker 12, and checks the relative position (for example, 45° in clockwise) of the position to which he/she wants to make the treatment tool 7 protrude (for example, position indicated by arrow in Fig. 5) with respect to the distal-end marker 12, and thus, it is possible for the operator to set, as the peripheral through-hole 9a through which the treatment tool 7 is inserted, the peripheral through-hole 9 that is disposed at a position shifted by an amount indicated by the above-described relative position with respect to the proximal-end marker 13 of the cap member 3. Then, after the peripheral through-hole 9a through which the treatment tool 7 is inserted is set, by inserting the treatment tool 7 from the set peripheral through-hole 9a, the operator can make the treatment tool 7 protrude at the desired position just by inserting the treatment tool 7 into the second lumen 5a by expanding the second lumen 5a connected to said peripheral through-hole 9a.

In other words, with the medical tube 1 according to this embodiment, even if the medical tube 1 is distorted with respect to the endoscope 6, because it is not necessary to alleviate the distortion, it is not necessary to apply torque to the medical tube 1 or to temporarily remove the medical tube 1 from the body to fix the distortion and then to reinsert the medical tube 1, and thus, there is an advantage in that it is possible to reduce the burden exerted on the patient.

Note that, regarding the distal-end marker 12 and the proximal-end marker 13 of the medical tube 1 according to this embodiment, there is no limitation to the above-described embodiment, and other arbitrary markers may be employed. For example, as shown in Figs. 6 and 7, identification information for identifying the second lumen 5a, for example, a serial number, may be employed as the distal-end marker 12 and the proximal-end marker 13.

In this case, from among the serial numbers indicated on the inner surface of the first lumen 4 in the image P acquired by using the endoscope 6, the operator reads the serial number (for example, "6") of the position at which he/she wants to make the treatment tool 7 protrude, and, by inserting the treatment tool 7 via the peripheral through-hole 9a for which the corresponding serial number (for example, "6") is provided on the cap member 3, he/she can make the treatment tool 7 protrude at the desired position in a simple manner. In other words, with such serial numbers, there is an advantage in that the operator can directly ascertain the position at which the treatment tool 7 should be inserted on the basis of the serial number he/she has read in the image P acquired by using the endoscope 6.

In this embodiment, although an example in which the second lumens 5 and 5a are collapsed and expanded when the treatment tool 7 is inserted has been described, there is no limitation thereto, and second lumens 5 and 5a that are not collapsed may be employed.

Although an example in which the cap member 3 is attached in order to facilitate insertion of the treatment tool 7 into one of the second lumens 5 and 5a has been described, there is no limitation thereto, and the cap member 3 may be omitted. In that case, the proximal-end marker 13 may be provided at the proximal-end portion of the tube main body 2.

Next, a medical system 15 according to an embodiment of the present invention will be described below with reference to the drawings.

In describing this embodiment, the same reference signs are assigned to portions that have common configurations with those of the medical tube 1 according to the above-described embodiment, and descriptions thereof will be omitted.

As shown in Fig. 8, the medical system 15 according to this embodiment is provided with: the medical tube 1; the endoscope 6 inserted into the first lumen 4 of the medical tube 1; a display portion 16 that displays the image P acquired by using the endoscope 6; an image-processing portion (image processor) 17 that recognizes the distal-end marker 12 in the acquired image P; and a notifying portion 18 that issues a notification about the identification information of the second lumen 5 on the basis of the distal-end marker 12 recognized by the image-processing portion 17.

Although the medical tube 1 has a structure similar to that of the above-described embodiment, as the distal-end marker 12, a straight-line marker is drawn on the inner surface of the first lumen 4 along the axial direction, as shown in Fig. 1, and, as the proximal-end marker 13, a set of identification information for identifying the individual second lumens 5 and 5a, for example, serial numbers, are drawn in the vicinity of the individual peripheral through-holes 9 and 9a of the cap member 3, as shown in Fig. 7.

As shown in Fig. 5, the image-processing portion 17 is configured so as to recognize an angle θ of the distal-end marker 12 in the image P acquired by using the endoscope 6.

Then, the notifying portion 18 displays the serial numbers of the second lumens 5 and 5a on the display portion 16 by superimposing them on the endoscope image P, as shown in Fig. 9, on the basis of the angle θ of the distal-end marker 12 recognized by the image-processing portion 17.

In other words, in the case in which the distal-end marker 12 is provided at a position aligned with the second lumen 5 having the serial number "1" and, as shown in Fig. 9, the serial numbers at the proximal-end marker 13 are assigned to the individual second lumens 5 and 5a clockwise when facing forward, the notifying portion 18 may display "1" in the vicinity of the recognized distal-end marker 12, as shown in Fig. 9, and may display as many serial numbers as there are the second lumens 5 and 5a clockwise centered on the image center with substantially equal intervals therebetween.

By doing so, the operator can easily check the peripheral through-hole 9a into which the treatment tool 7 should be inserted on the basis of the serial number displayed on the image P. Thus, even if the medical tube 1 is distorted with respect to the endoscope 6, it is possible to make the treatment tool 7 protrude at the desired position in the image P and to perform treatment just by inserting the treatment tool 7 into the checked peripheral through-hole 9a.

Note that, in the case in which the position in the image P at which the treatment tool 7 should be made to protrude is set in advance, instead of displaying the serial numbers corresponding to all of the second lumens 5 and 5a in the image P, the notifying portion 18 may issue a notification about only the serial number (for example, "2") of the second lumen 5a into which the treatment tool 7 should be inserted, as shown in Fig. 10. In addition to the case in which a notification is issued about a single serial number, a notification may be issued about serial numbers of two or more optimal second lumens 5a.

For example, in the case in which the treatment tool 7 is a manipulator of an isomorphic master-slave system, the serial numbers of the optimal second lumens 5a may be selected so that the positional relationship between the eyes of the operator and a manipulating portion (not shown) becomes equal to the positional relationship between the endoscope 6 and the distal-end portion of the treatment tool 7.

In the case in which there are multiple types of treatment tools 7 to be inserted, and the optimal protruding positions in the image P differ in accordance with the treatment tools 7, an input portion 19 with which input about the types of the treatment tools 7 is made may be provided, as shown in Fig. 11.

In other words, on the basis of the angle θ of the distal-end marker 12 recognized by the image-processing portion 17 and in accordance with the type of the treatment tool 7 input via the input portion 19, the notifying portion 18 may set and issue a notification about the peripheral through-hole 9a through which the treatment tool 7 should be inserted.

For example, in the case in which the treatment tool 7 is a gripping forceps for the lesion-raising, it is preferable that the treatment tool 7 be protruded at a position away from the lesion. In this case, the position of the lesion may be specified by means of image recognition, alternatively, by allowing the operator to specify the position in the image P, and thus, the second lumen 5a at a position that is the farthest from said lesion may be selected.

In the case in which the treatment tool 7 is a joint-less treatment tool, because the lesion needs to be in the vicinity of a position extended straight from the distal-end of the opening 14 of the medical tube 1, the second lumen 5a that satisfies such conditions may be selected.

In the case in which another treatment tool 7 is in use, the serial numbers of the second lumens 5 and 5a may be set so that the treatment tool 7 that is newly inserted does not interfere with the treatment tool 7 in use. Examples of such cases include cases in which the need to insert a new treatment tool 7 unexpectedly arises, such as when bleeding occurs, when performing an additional local injection, or the like.

In this embodiment, although the notifying portion 18 displays the serial numbers for identifying the second lumens 5 and 5a on the display portion 16 together with the image P, alternatively, light emitting portions such as LEDs or the like may be provided in the cap member 3 at positions corresponding to the individual second lumens 5 and 5a, and a notification may be issued by causing an LED corresponding to the selected second lumen 5a to emit light. In addition, shutters that open and close the individual peripheral through-holes 9 and 9a may be provided, and a notification may be issued by opening only the shutter of the peripheral through-hole 9a corresponding to the selected second lumen 5a.

### {Reference Signs List}

- 1: medical tube
- 2: tube main body
- 3: cap member
- 4: first lumen
- 5, 5a: second lumen
- 6: endoscope
- 7: treatment tool
- 9, 9a: peripheral through-hole (proximal-end opening)
- 10: insertion cylindrical portion
- 11: proximal-end opening
- 12: distal-end marker (first indicator)
- 13: proximal-end marker (second indicator)
- 14: distal-end opening
- 15: medical system
- 16: display portion
- 17: image-processing portion
- 18: notifying portion
- 19: input portion
- P: image

## Claims

1. A medical tube (1) comprising:
a tube main body (2), which is constituted of a material possessing flexibility,
a first lumen (4) that forms an endoscope channel by penetrating through the tube main body in a longitudinal-axis direction;
a plurality of second lumens (5, 5a) that are capable of forming treatment-tool channels by penetrating through the tube main body in the longitudinal-axis direction at a periphery of the first lumen with spacings therebetween in a circumferential direction; **characterized by**
a first indicator (12) that is provided on an inner surface in the vicinity of a distal end of the first lumen and that specifies a circumferential-direction position of any one of the second lumens with respect to the first lumen; and
a second indicator (13) that is provided at a proximal end of the tube main body, and that indicates the second lumen specified by the first indicator.

2. A medical tube according to Claim 1, wherein the second lumens are provided so that inner diameters thereof can be expanded in directions in which an outer diameter of the tube main body would be increased.

3. A medical tube according to Claim 1, wherein the first indicators are identification information of the individual second lumens with which the second indicators corresponding thereto can be identified.

4. A medical tube (1) comprising:
a tube main body (2) that is constituted of a material possessing flexibility;
a cap member (3) that is attached to the proximal end of the tube main body;
a first lumen (4) that forms an endoscope channel by penetrating through the tube main body in a longitudinal-axis direction;
a plurality of second lumens (5, 5a) that are capable of forming treatment-tool channels by penetrating through the tube main body in the longitudinal-axis direction at a periphery of the first lumen with spacings therebetween in a circumferential direction; **characterized by**
a first indicator (12) that is provided on an inner surface in the vicinity of a distal end of the first lumen and that specifies a circumferential-direction position of any one of the second lumens with respect to the first lumen; and
a second indicator (13) that is provided on the cap member, and that indicates the second lumen specified by the first indicator.

5. A medical tube according to Claim 4,
wherein the cap member has a large enough diameter to allow a treatment tool to be inserted thereinto, and is provided with a plurality of insertion cylindrical portions that are inserted into proximal-end openings of the individual second lumens and expand the proximal-end openings.

6. A medical tube according to Claim 4, wherein the second lumens are provided so that inner diameters thereof can be expanded in directions in which an outer diameter of the tube main body would be increased.

7. A medical tube according to Claim 4, wherein the first indicators are identification information of the individual second lumens with which the second indicators corresponding thereto can be identified.

8. A medical system comprising:
a medical tube according to Claim 1 or 4;
an endoscope that is inserted into the first lumen of the medical tube;
a display portion that displays an image acquired by using the endoscope;
an image-processing portion that recognizes the first indicator in the image; and
a notifying portion that issues, on the basis of the first indicator recognized by the image-processing portion, a notification about the identification information of one or more of the second lumens specified by the first indicator.

9. A medical system according to Claim 8, wherein the notifying portion displays, in the image displayed on the display portion, the identification information in association with positions of distal-end openings of one or more of the second lumens.

10. A medical system according to Claim 8, wherein the notifying portion issues, on the basis of the first indicator recognized by the image-processing portion, a notification about the identification information of one or more of the second lumens to be used as the treatment-tool channels.

11. A medical system according to Claim 10, further comprising:
an input portion with which information about a treatment tool to be used is input,
wherein the notifying portion issues, on the basis of the information about the treatment tool input via the input portion, a notification about the identification information of one or more of the second lumens that are suitable to be used as the treatment-tool channels.

12. A medical system according to Claim 10, wherein the notifying portion issues a notification by making the second indicators corresponding to the second lumens to be used as the treatment-tool channels differ from the rest of the second indicators at the proximal end of the tube main body.

## Patentansprüche

1. Medizinischer Tubus (1), der umfasst:
einen Tubushauptkörper (2), der aus einem flexiblen Material gebildet ist,
ein erstes Lumen (4), das einen Endoskopkanal bildet, indem es den Tubushauptkörper in einer Längsachsenrichtung durchdringt;
eine Mehrzahl von zweiten Lumen (5, 5a), die in der Lage sind, Behandlungswerkzeugkanäle zu bilden, indem sie den Tubushauptkörper in Längsachsenrichtung an einer Peripherie des ersten Lumens mit Zwischenräumen dazwischen in einer Umfangsrichtung durchdringen; **gekennzeichnet durch**:
einen ersten Indikator (12), der auf einer Innenfläche in der Nähe eines distalen Endes des ersten Lumens bereitgestellt ist und der eine Umfangsrichtungsposition eines beliebigen der zweiten Lumen in Bezug auf das erste Lumen spezifiziert; und
einen zweiten Indikator (13), der an einem proximalen Ende des Tubushauptkörpers bereitgestellt ist und das zweite Lumen indiziert, das durch den ersten Indikator spezifiziert wird.

2. Medizinischer Tubus nach Anspruch 1, wobei die zweiten Lumen so bereitgestellt sind, dass ihre Innendurchmesser in Richtungen erweitert werden können, in die ein Außendurchmesser des Tubushauptkörpers erhöht werden würde.

3. Medizinischer Tubus nach Anspruch 1, wobei die ersten Indikatoren Identifikationsinformationen der einzelnen zweiten Lumen sind, mit denen die diesen entsprechenden zweiten Indikatoren identifiziert werden können.

4. Medizinischer Tubus (1), der umfasst:
einen Tubushauptkörper (2), der aus einem flexiblen Material gebildet ist;
ein Kappenelement (3), das am proximalen Ende des Tubushauptkörpers angebracht ist;
ein erstes Lumen (4), das einen Endoskopkanal bindet, indem es den Tubushauptkörper in einer Längsachsenrichtung durchdringt;
eine Mehrzahl von zweiten Lumen (5, 5a), die in der Lage sind, Behandlungswerkzeugkanäle zu bilden, indem sie den Tubushauptkörper in Längsachsenrichtung an einer Peripherie des ersten Lumens mit Zwischenräumen dazwischen in einer Umfangsrichtung durchdringen; **gekennzeichnet durch**:
einen ersten Indikator (12), der auf einer Innenfläche in der Nähe eines distalen Endes des ersten Lumens bereitgestellt ist und der eine Umfangsrichtungsposition eines beliebigen der zweiten Lumen in Bezug auf das erste Lumen spezifiziert; und
einen zweiten Indikator (13), der auf dem Kappenelement bereitgestellt ist und das zweite Lumen indiziert, das durch den ersten Indikator spezifiziert wird.

5. Medizinischer Tubus nach Anspruch 4,
wobei das Kappenelement einen Durchmesser aufweist, der groß genug ist, dass er das Einführen eines Behandlungswerkzeugs ermöglicht, und mit einer Mehrzahl von zylindrischen Einführabschnitten versehen ist, die in proximalendige Öffnungen der einzelnen zweiten Lumen eingeführt werden und die proximalendigen Öffnungen erweitern.

6. Medizinischer Tubus nach Anspruch 4, wobei die zweiten Lumen so bereitgestellt sind, dass ihre Innendurchmesser in Richtungen erweitert werden können, in denen ein Außendurchmesser des Tubushauptkörpers erhöht werden würde.

7. Medizinischer Tubus nach Anspruch 4, wobei die ersten Indikatoren Identifikationsinformationen der einzelnen zweiten Lumen sind, mit denen die diesen entsprechenden zweiten Indikatoren identifiziert werden können.

8. Medizinisches System, das umfasst:
einen medizinischen Tubus nach Anspruch 1 oder 4;
ein Endoskop, das in das erste Lumen des medizinischen Tubus eingeführt wird;
einen Anzeigeabschnitt, der ein unter Verwendung des Endoskop erfasstes Bild anzeigt;
einen Bildverarbeitungsabschnitt, der den ersten Indikator auf dem Bild erkennt; und
einen Benachrichtigungsabschnitt, der eine Benachrichtigung über die Identifikationsinformation eines oder mehrerer der zweiten Lumen, die durch den ersten Indikator spezifiziert werden, auf Basis des vom Bildverarbeitungsabschnitt erkannten ersten Indikators ausgibt.

9. Medizinisches System nach Anspruch 8, wobei der Benachrichtigungsabschnitt die Identifikationsinformationen in Assoziation mit Positionen distalendiger Öffnungen eines oder mehrerer der zweiten Lumen auf dem Bild anzeigt, das auf dem Anzeigeabschnitt angezeigt wird.

10. Medizinisches System nach Anspruch 8, wobei der Benachrichtigungsabschnitt eine Benachrichtigung über die Identifikationsinformationen eines oder mehrerer zweiter Lumen, die als Behandlungswerkzeugkanäle zu verwenden sind, auf Basis des vom Bildverarbeitungsabschnitt erkannten ersten Indikators ausgibt.

11. Medizinisches System nach Anspruch 10, das ferner umfasst:
einen Eingabeabschnitt, mit dem Informationen zu einem zu verwendeten Behandlungswerkzeug eingegeben werden,
wobei der Benachrichtigungsabschnitt eine Benachrichtigung über die Identifikationsinformationen eines oder mehrerer der zweiten Lumen, die sich für eine Verwendung als Behandlungswerkzeugkanäle eignen, auf Basis der Informationen zum Behandlungswerkzeug ausgibt, die über den Eingabeabschnitt eingegeben werden.

12. Medizinisches System nach Anspruch 10, wobei der Benachrichtigungsabschnitt eine Benachrichtigung ausgibt, indem er bewirkt, dass sich die zweiten Indikatoren, die den zweiten Lumen entsprechen, die als Behandlungswerkzeugkanäle zu verwenden sind, von den restlichen zweiten Indikatoren am proximalen Ende des Tubushauptkörpers unterscheiden.

## Revendications

1. Tube médical (1) comprenant :
un corps principal de tube (2), qui est constitué d'un matériau possédant une flexibilité,
une première lumière (4) qui forme un canal d'endoscope par pénétration à travers le corps principal de tube dans une direction d'axe longitudinal ;
une pluralité de secondes lumières (5, 5a) qui sont aptes à former des canaux d'outil de traitement par pénétration à travers le corps principal de tube dans la direction d'axe longitudinal au niveau d'une périphérie de la première lumière, avec des espacements entre elles dans une direction circonférentielle ;
**caractérisé par**
un premier indicateur (12) qui est disposé sur une surface interne au voisinage d'une extrémité distale de la première lumière, et qui spécifie une position de direction circonférentielle de l'une quelconque des secondes lumières par rapport à la première lumière ; et
un second indicateur (13) qui est disposé au niveau d'une extrémité proximale du corps principal de tube, et qui indique la seconde lumière spécifiée par le premier indicateur.

2. Tube médical selon la revendication 1, dans lequel les secondes lumières sont disposées de telle sorte que leurs diamètres internes peuvent être étendus dans des directions dans lesquelles un diamètre externe du corps principal de tube serait augmenté.

3. Tube médical selon la revendication 1, dans lequel les premiers indicateurs sont des informations d'identification des secondes lumières individuelles avec lesquelles les seconds indicateurs correspondant à celles-ci peuvent être identifiés.

4. Tube médical (1) comprenant :
un corps principal de tube (2) qui est constitué d'un matériau possédant une flexibilité ;
un élément de capuchon (3) qui est fixé à l'extrémité proximale du corps principal de tube ;
une première lumière (4) qui forme un canal d'endoscope par pénétration à travers le corps principal de tube dans une direction d'axe longitudinal ;
une pluralité de secondes lumières (5, 5a) qui sont aptes à former des canaux d'outil de traitement par pénétration à travers le corps principal de tube dans la direction d'axe longitudinal au niveau d'une périphérie de la première lumière, avec des espacements entre elles dans une direction circonférentielle ;
**caractérisé par**
un premier indicateur (12) qui est disposé sur une surface interne au voisinage d'une extrémité distale de la première lumière, et qui spécifie une position de direction circonférentielle de l'une quelconque des secondes lumières par rapport à la première lumière ; et
un second indicateur (13) qui est disposé sur l'élément de capuchon, et qui indique la seconde lumière spécifiée par le premier indicateur.

5. Tube médical selon la revendication 4, dans lequel l'élément de capuchon a un diamètre assez grand pour permettre à un outil de traitement d'être inséré dans celui-ci, et comprend une pluralité de parties cylindriques d'insertion qui sont insérées dans des ouvertures d'extrémité proximale des secondes lumières individuelles et étendent les ouvertures d'extrémité proximale.

6. Tube médical selon la revendication 4, dans lequel les secondes lumières sont disposées de telle sorte que leurs diamètres internes peuvent être étendus dans des directions dans lesquelles un diamètre externe du corps principal de tube serait augmenté.

7. Tube médical selon la revendication 4, dans lequel les premiers indicateurs sont des informations d'identification des secondes lumières individuelles avec lesquelles les seconds indicateurs correspondant à celles-ci peuvent être identifiés.

8. Système médical comprenant :
un tube médical selon la revendication 1 ou 4 ;
un endoscope qui est inséré dans la première lumière du tube médical ;
une partie d'affichage qui affiche une image acquise à l'aide de l'endoscope ;
une partie de traitement d'image qui reconnaît le premier indicateur dans l'image ; et
une partie de notification qui délivre, sur la base du premier indicateur reconnu par la partie de traitement d'image, une notification concernant les informations d'identification d'une ou plusieurs des secondes lumières spécifiées par le premier indicateur.

9. Système médical selon la revendication 8, dans lequel la partie de notification affiche, dans l'image affichée sur la partie d'affichage, les informations d'identification en association avec des positions d'ouvertures d'extrémité distale d'une ou plusieurs des secondes lumières.

10. Système médical selon la revendication 8, dans lequel la partie de notification délivre, sur la base du premier indicateur reconnu par la partie de traitement d'image, une notification concernant les informations d'identification d'une ou plusieurs des secondes lumières à utiliser comme canaux d'outil de traitement.

11. Système médical selon la revendication 10, comprenant en outre :
une partie d'entrée avec laquelle des informations concernant un outil de traitement à utiliser sont entrées,
la partie de notification délivrant, sur la base des informations concernant l'outil de traitement entrées par l'intermédiaire de la partie d'entrée, une notification concernant les informations d'identification d'une ou plusieurs des secondes lumières qui sont appropriées pour être utilisées comme canaux d'outil de traitement.

12. Système médical selon la revendication 10, dans lequel la partie de notification délivre une notification en amenant les seconds indicateurs correspondant aux secondes lumières à utiliser comme canaux d'outil de traitement à être différents du reste des seconds indicateurs au niveau de l'extrémité proximale du corps principal de tube.
